# EUROPÄISCHE PATENTANMELDUNG

(11) **EP 4 592 272 A1**
(43) Veröffentlichungstag der Anmeldung: **30.07.2025**
(21) Anmeldenummer: 24153478.3
(22) Anmeldetag: 23.01.2024
(51) Int. Cl.: C07C 67/00, C07C 69/24, C07C 51/09, C07C 53/126

(54) **VERFAHREN ZUR UMSETZUNG EINES ETHERS ZUM ENTSPRECHENDEN ESTER**

(71) Anmelder: Evonik Oxeno GmbH & Co. KG, 45772 Marl (DE)
(72) Erfinder: HUANG, Weiheng, Waco, TX 76706 (US); JACKSTELL, Ralf, 18106 Rostock (DE); BELLER, Matthias, 18211 Ostseebad Nienhagen (DE); FRANKE, Robert, 45772 Marl (DE)
(74) Vertreter: Evonik Patent Association

(57) **Zusammenfassung**

Verfahren zur Umsetzung eines Ethers zum entsprechenden Ester.

## Beschreibung

Die vorliegende Erfindung betrifft ein Verfahren zur Umsetzung eines Ethers zum entsprechenden Ester.

Caprylsäure (n-Octansäure) ist eine wichtige industrielle Verbindung mit vielfältigen Anwendungsmöglichkeiten, wie zum Beispiel in der Lebensmittelindustrie oder der Pharmazie.

Bei der chemischen Herstellung kann, im Gegensatz zu der Gewinnung aus pflanzlichen Produkten, reine n-Octansäure gewonnen werden. Dies erfolgt klassischer Weise durch Telomerisierung von 1,3-Butadien mit Wasser zu 2,7-Octadienol. Den ungesättigte Aldehyd erhält man durch Isomerisierung des Alkohols. Durch Hydrierung von 7-Octenal zu Octanal und anschließender Oxidation des Octanals erhält man letztendlich die gewünschte n-Octansäure.

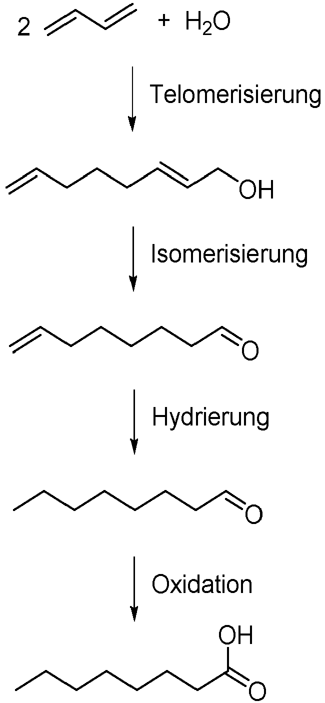

Dieser mehrstufige Prozess führt zu einer großen Menge an Nebenprodukten und ist recht energieaufwendig.

Aufgabe der vorliegenden Erfindung war es, Teilschritte, der zuvor beschriebenen Syntheseroute zur Herstellung von Säuren, zu optimieren. Dies soll zu einer effizienteren Syntheseroute beitragen.

Die Aufgabe wird gelöst durch ein Verfahren nach Anspruch 1.

Verfahren umfassend die Verfahrensschritte:
a) Vorlegen eines Ethers mit 5 bis 30 Kohlenstoffatomen, welcher mindestens zwei Doppelbindungen aufweist;
b) Zugabe eines Liganden gemäß Formel (I): 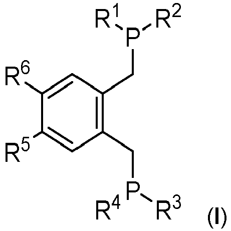 wobei
   R¹, R², R³, R⁴ jeweils für -(C₁-C₁₂)-Alkyl stehen,
   R⁵, R⁶ jeweils für einen Rest stehen ausgewählt aus: -H, -O-(C₁-C₁₂)-Alkyl, -(C₁-C₁₂)-Alkyl,
   und einer Verbindung, welche Pd umfasst;
c) Zugabe einer Lewis-Säure;
d) Erwärmen des Reaktionsgemisches aus a) bis c), wobei der Ether zu einem Ester umgesetzt wird, und der Ester die gleiche Anzahl an Kohlenstoffatomen aufweist wie der Ether.

Der Begriff (C₁-C₁₂)-Alkyl umfasst geradkettige und verzweigte Alkylgruppen mit 1 bis 12 Kohlenstoffatomen.

Durch das erfindungsgemäße Verfahren kann Caprylsäure auf einem deutlich effizienteren Weg hergestellt werden:

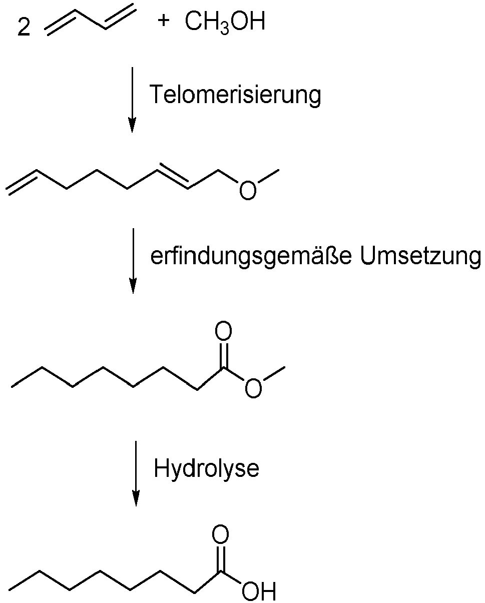

In einer Variante des Verfahrens weist der Ether die Formel (II) auf: 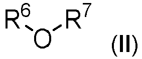 und
R⁶ für eine Kohlenstoffkette steht, welche mindesten vier C-Atome aufweist und mindestens zwei Doppelbindungen aufweist;
R⁷ für einen Kohlenstoffkette steht, welche mindesten ein C-Atom aufweist.

In einer Variante des Verfahrens steht R⁷ für einen Kohlenstoffkette mit 1 bis 4 C-Atomen.

In einer Variante des Verfahrens steht R⁶ für einen Kohlenstoffkette mit 5 bis 12 C-Atomen.

In einer Variante des Verfahrens steht R⁶ für einen Kohlenstoffkette, welche genau zwei Doppelbindung aufweist.

In einer Variante des Verfahrens weist der Ether die Struktur (2) auf:

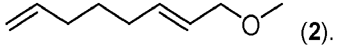

In einer Variante des Verfahrens wird der Ether durch Telomerisierung von 1,3-Butadien gewonnen.

In einer Variante des Verfahrens wird der Ether durch Telomerisierung von 1,3-Butadien mit Methanol gewonnen.

In einer Variante des Verfahrens stehen R⁵, R⁶ für -H.

In einer Variante des Verfahrens stehen R¹, R², R³, R⁴ für -(C₁-C₄)-Alkyl.

In einer Variante des Verfahrens stehen R¹, R², R³, R⁴ für *-*^{t}Bu.

In einer Variante des Verfahrens weist der Ligand die Struktur (1) auf:

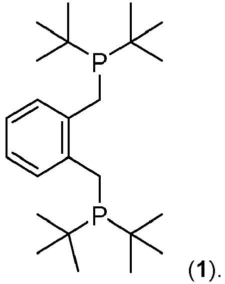

In einer Variante des Verfahrens ist die Verbindung in Verfahrensschritt b), welche Pd umfasst, ausgewählt aus: Palladiumdichlorid, Palladiumdibromid, Palladiumdiiodid, Palladium(II)-acetylacetonat, Palladium(II)-acetat, Dichloro(1,5-cyclooctadiene)palladium(II), Bis(dibenzylideneaceton)palladium, Bis(acetonitrile)dichloropalladium(II), Palladium(cinnamyl)dichlorid.

In einer Variante des Verfahrens ist die Verbindung in Verfahrensschritt b), welche Pd umfasst, ausgewählt aus: Pd(dba)₂, Pd(OAc)₂, PdI₂.

In einer Variante des Verfahrens ist die Lewis-Säure ausgewählt aus: Al(OTf)₃, Ga(OTf)₃, In(OTf)₃, Bi(OTf)₃, Fe(OTf)₃, Al(OMs)₃, Al(OTs)₃.

In einer Variante des Verfahrens liegt die Lewis-Säure als sulfoniertes Tetrafluorethylen-Polymer vor.

In einer Variante des Verfahrens erfolgt das Erwärmen in Verfahrensschritt d) auf eine Temperatur im Bereich von 70 °C bis 140 °C.

In einer Variante des Verfahrens erfolgt das Erwärmen in Verfahrensschritt d) auf eine Temperatur im Bereich von 80 °C bis 120 °C.

In einer Variante des Verfahrens umfasst das Verfahren den zusätzlichen Verfahrensschritt c'): c') Zugabe eines Lösungsmittels.

In einer Variante des Verfahrens ist das Lösungsmittel Methanol.

In einer Variante des Verfahrens umfasst das Verfahren den zusätzlichen Verfahrensschritt e):
e) Hydrolyse des in d) erhaltenen Esters zur Säure.

Im Folgenden soll die Erfindung anhand von Ausführungsbeispielen näher erläutert werden.

Die Versuche wurde mit 1,0 mmol 1-MODE (**2**) in einer Methanollösung (2,0 mL) durchgeführt. Es werden Pd(dba)₂ (dba: Dibenzylidenaceton) als Vorläufer, 1,2-Bis(di-tert-butylphosphinomethyl)benzol (dₜbpx) (**1**) als Ligand und Al(OTf)₃ (OTf: Trifluormethansulfonat) als Lewis-Säure eingesetzt. Es wurde ein N₂-Druck von 40 bar verwendet. Die Reaktion lief über 6 Stunden bei 100 °C.

### Reaktionsbedingungen:

Pd(dba)₂ 0,5 mol%, Ligand (1) 1,0 mol%, Al(OTf)₃ 2,0 mol%, MeOH, N₂ 40 bar, T: 100 °C, t: 6 h

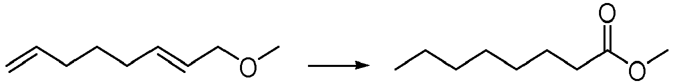

Die Ausbeute an Ester betrug 85 %.

Der Versuch wurde in abgeänderten Variationen durchgeführt:
- ohne Al(OTf)₃: keine Ausbeute
- ohne Pd(dba)₂: keine Ausbeute

### Variation der Pd-Verbindung

Der Versuch wurde mit unterschiedlichen Pd-Verbindungen durchgeführt. Die Ergebnisse sind in der nachfolgenden Tabelle zusammengestellt:

**Tabelle 1:**

| Pd-Verbindung | Ausbeute |
|---|---|
| Pd(dba)₂ | 85 % |
| Pd(OAc)₂ | 54 % |
| PdI₂ | 92 % |

### Variation der Lewis-Säure

Der Versuch wurde mit unterschiedlichen Lewis-Säuren durchgeführt.

Die Ergebnisse sind in der nachfolgenden Tabelle zusammengestellt.

### Reaktionsbedingungen:

Pd(dba)₂ 0,5 mol%, Ligand (**1**) 1,0 mol%, Lewis-Säure 2,0 mol%, MeOH, N₂ 40 bar, T: 100 °C, t: 20 h

**Tabelle 2:**

| Lewis-Säure | Ausbeute |
|---|---|
| Ga(OTf)₃ | 96 % |
| In(OTf)₃ | 95 % |
| Bi(OTf)₃ | 90 % |
| Fe(OTf)₃ | 92 % |
| Al(OMs)₃ | 97 % |
| Al(OTs)₃ | 96 % |

### Vergleichsversuch mit einer Brønsted-Säure

### Reaktionsbedingungen:

Pd(dba)₂ 0,5 mol%, Ligand (**1**) 1,0 mol%, Säure 2,0 mol%, MeOH, N₂ 40 bar, T: 100 °C, t: 6 h

**Tabelle 3:**

| Säure | Ausbeute |
|---|---|
| Al(OTf)₃ (Lewis-Säure) | 85 % |
| PTSA·H₂O (Br⌀nsted-Säure)* | 28 % |

| | |
|---|---|
| * nicht erfindungsgemäßer Vergleichsversuch | |

Wie die durchgeführten Versuche zeigen, wird die Aufgabe durch ein erfindungsgemäßes Verfahren gelöst.

## Patentansprüche

1. Verfahren umfassend die Verfahrensschritte:
a) Vorlegen eines Ethers mit 5 bis 30 Kohlenstoffatomen, welcher mindestens zwei Doppelbindungen aufweist;
b) Zugabe eines Liganden gemäß Formel (**I**): 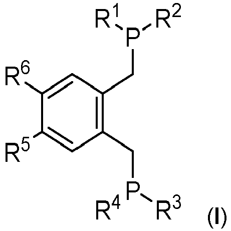 wobei
R¹, R², R³, R⁴ jeweils für -(C₁-C₁₂)-Alkyl stehen,
R⁵, R⁶ jeweils für einen Rest stehen ausgewählt aus: -H, -O-(C₁-C₁₂)-Alkyl, -(C₁-C₁₂)-Alkyl,
und einer Verbindung, welche Pd umfasst;
c) Zugabe einer Lewis-Säure;
d) Erwärmen des Reaktionsgemisches aus a) bis c), wobei der Ether zu einem Ester umgesetzt wird, und der Ester die gleiche Anzahl an Kohlenstoffatomen aufweist wie der Ether.

2. Verfahren nach Anspruch 1,
wobei der Ether die Formel (**II**) aufweist: 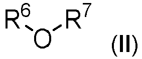 und
R⁶ für eine Kohlenstoffkette steht, welche mindesten vier C-Atome aufweist und mindestens zwei Doppelbindungen aufweist;
R⁷ für einen Kohlenstoffkette steht, welche mindesten ein C-Atom aufweist.

3. Verfahren nach Anspruch 2,
wobei R⁷ für einen Kohlenstoffkette mit 1 bis 4 C-Atomen steht.

4. Verfahren nach einem der Ansprüche 2 oder 3,
wobei R⁶ für einen Kohlenstoffkette mit 5 bis 12 C-Atomen steht.

5. Verfahren nach einem der Ansprüche 2 bis 4,
wobei R⁶ für einen Kohlenstoffkette steht, welche genau zwei Doppelbindung aufweist.

6. Verfahren nach einem der Ansprüche 1 bis 5,
wobei der Ether die Struktur (**2**) aufweist:

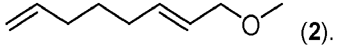

7. Verfahren nach einem der Ansprüche 1 bis 6,
wobei R⁵, R⁶ für -H stehen.

8. Verfahren nach einem der Ansprüche 1 bis 7,
wobei der Ligand die Struktur (**1**) aufweist:

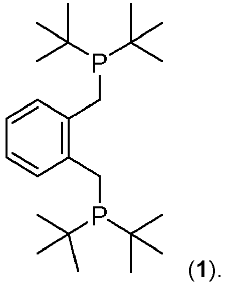

9. Verfahren nach einem der Ansprüche 1 bis 8,
wobei die Verbindung in Verfahrensschritt b), welche Pd umfasst, ausgewählt ist aus:
Palladiumdichlorid, Palladiumdibromid, Palladiumdiiodid, Palladium(II)-acetylacetonat, Palladium(II)-acetat, Dichloro(1,5-cyclooctadiene)palladium(II), Bis(dibenzylideneaceton) palladium, Bis(acetonitrile)dichloropalladium(II), Palladium(cinnamyl)dichlorid.

10. Verfahren nach einem der Ansprüche 1 bis 9,
wobei die Verbindung in Verfahrensschritt b), welche Pd umfasst, ausgewählt ist aus: Pd(dba)₂, Pd(OAc)₂, PdI₂.

11. Verfahren nach einem der Ansprüche 1 bis 10,
wobei die Lewis-Säure ausgewählt ist aus: Al(OTf)₃, Ga(OTf)₃, In(OTf)₃, Bi(OTf)₃, Fe(OTf)₃, Al(OMs)ₐ, Al(OTs)₃.

12. Verfahren nach einem der Ansprüche 1 bis 11,
wobei das Erwärmen in Verfahrensschritt d) auf eine Temperatur erfolgt, im Bereich von 70 °C bis 140 °C.

13. Verfahren nach einem der Ansprüche 1 bis 12,
wobei das Verfahren den zusätzlichen Verfahrensschritt c') umfasst:
c') Zugabe eines Lösungsmittels.

14. Verfahren nach einem der Ansprüche 1 bis 13,
wobei das Verfahren den zusätzlichen Verfahrensschritt e) umfasst:
e) Hydrolyse des in d) erhaltenen Esters zur Säure.

## Geänderte Patentansprüche

### Geänderte Patentansprüche gemäss Regel 137(2) EPÜ.

1. Verfahren umfassend die Verfahrensschritte:
a) Vorlegen eines Ethers mit 5 bis 30 Kohlenstoffatomen, welcher mindestens zwei Doppelbindungen aufweist,
wobei der Ether die Formel (II) aufweist: 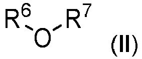 und
R⁶ für eine Kohlenstoffkette steht, welche mindesten vier C-Atome aufweist und mindestens zwei Doppelbindungen aufweist,
R⁷ für einen Kohlenstoffkette steht, welche mindesten ein C-Atom aufweist;
b) Zugabe eines Liganden gemäß Formel (I): 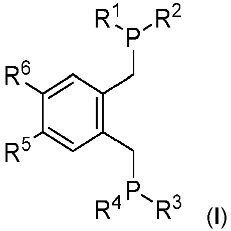 wobei
R¹, R², R³, R⁴ jeweils für -(C₁-C₁₂)-Alkyl stehen,
R⁵, R⁶ jeweils für einen Rest stehen ausgewählt aus: -H, -O-(C₁-C₁₂)-Alkyl, -(C₁-C₁₂)-Alkyl,
und einer Verbindung, welche Pd umfasst;
c) Zugabe einer Lewis-Säure;
c') Zugabe von Methanol;
d) Erwärmen des Reaktionsgemisches aus a) bis c'), wobei der Ether zu einem Ester umgesetzt wird, und der Ester die gleiche Anzahl an Kohlenstoffatomen aufweist wie der Ether.

2. Verfahren nach Anspruch 1,
wobei R⁷ für einen Kohlenstoffkette mit 1 bis 4 C-Atomen steht.

3. Verfahren nach einem der Ansprüche 1 oder 2,
wobei R⁶ für einen Kohlenstoffkette mit 5 bis 12 C-Atomen steht.

4. Verfahren nach einem der Ansprüche 1 bis 3,
wobei R⁶ für einen Kohlenstoffkette steht, welche genau zwei Doppelbindung aufweist.

5. Verfahren nach einem der Ansprüche 1 bis 4,
wobei der Ether die Struktur (2) aufweist:

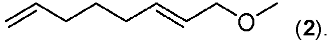

6. Verfahren nach einem der Ansprüche 1 bis 5, wobei R⁵, R⁶ für -H stehen.

7. Verfahren nach einem der Ansprüche 1 bis 6, wobei der Ligand die Struktur (1) aufweist:

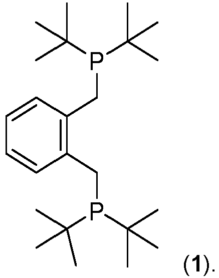

8. Verfahren nach einem der Ansprüche 1 bis 7,
wobei die Verbindung in Verfahrensschritt b), welche Pd umfasst, ausgewählt ist aus: Palladiumdichlorid, Palladiumdibromid, Palladiumdiiodid, Palladium(II)-acetylacetonat, Palladium(II)-acetat, Dichloro(1,5-cyclooctadiene)palladium(II), Bis(dibenzylideneaceton) palladium, Bis(acetonitrile)dichloropalladium(II), Palladium(cinnamyl)dichlorid.

9. Verfahren nach einem der Ansprüche 1 bis 8,
wobei die Verbindung in Verfahrensschritt b), welche Pd umfasst, ausgewählt ist aus: Pd(dba)₂, Pd(OAc)₂, PdI₂.

10. Verfahren nach einem der Ansprüche 1 bis 9,
wobei die Lewis-Säure ausgewählt ist aus: Al(OTf)₃, Ga(OTf)₃, In(OTf)₃, Bi(OTf)₃, Fe(OTf)₃, Al(OMs)₃, Al(OTs)₃.

11. Verfahren nach einem der Ansprüche 1 bis 10,
wobei das Erwärmen in Verfahrensschritt d) auf eine Temperatur erfolgt, im Bereich von 70 °C bis 140 °C.

12. Verfahren nach einem der Ansprüche 1 bis 11,
wobei das Verfahren den zusätzlichen Verfahrensschritt e) umfasst:
e) Hydrolyse des in d) erhaltenen Esters zur Säure.
